# EUROPEAN PATENT APPLICATION

(11) **EP 3 111 856 A1**
(43) Date of publication of application: **04.01.2017**
(21) Application number: 14884233.9
(22) Date of filing: 21.05.2014
(51) Int. Cl.: A61B 17/11

(54) **MEDICAL DEGRADABLE MAGNESIUM ALLOY MULTIPURPOSE ANASTOMOTIC PIECE**

(30) Priority: 26.02.2014 CN 201410068125
(71) Applicant: Dongguan Eontec Co., Ltd., Dongguan, Guangdong 523000 (CN)
(72) Inventor: LI, Yangde, Dongguan, Guangdong 523000 (CN)
(74) Representative: Hryszkiewicz, Danuta
(86) International application number: PCT/CN2014/077955
(87) International publication number: WO 2015/127723

(57) **Abstract**

A medical degradable magnesium alloy multipurpose anastomotic piece comprises an anastomotic piece substrate (1) made of magnesium alloy material; a plurality of turnup spines (2) arranged in a staggered and opposite mode are formed on the surface of the anastomotic piece substrate (1); the surface of the anastomotic piece substrate (1) and the surfaces of the turnup spines (2) are uniformly covered with a drug layer (3) which can be absorbed by a human body. The anastomotic piece substrate (1) with the plurality of turnup spines (2) can be rolled inwards or outwards to form a tubular shape for anastomosis of human tissues and organs. The rolled tubular anastomotic piece substrate (1) will be degraded or absorbed by the human body along with the slow healing of a wound.

## Description

The invention belongs to the field of medical apparatus and instruments, and relates to an assistive device of medical stapling device, more particularly to a degradable surgical staple made from magnesium.

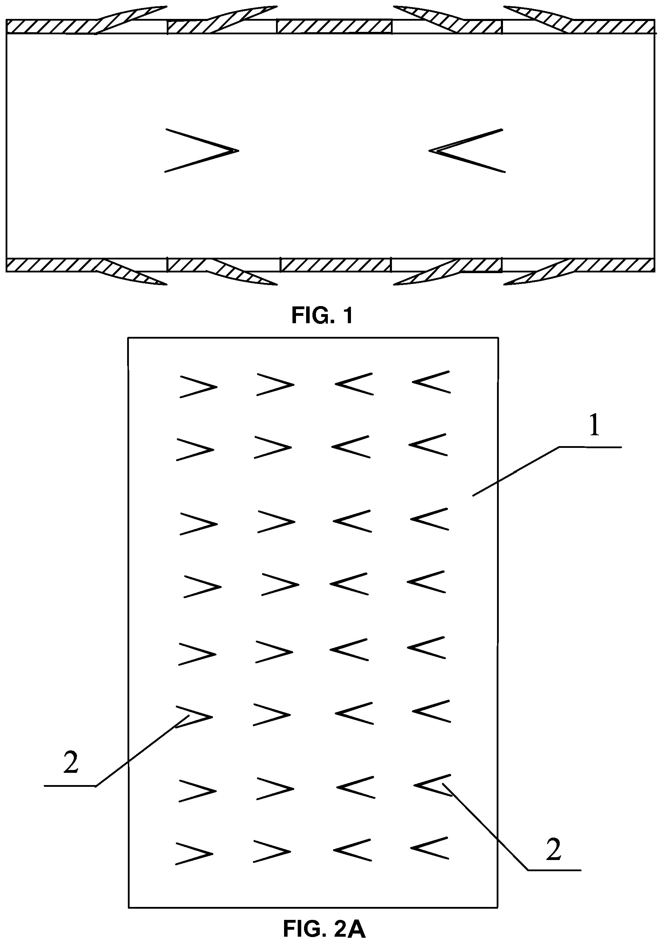

Medical science and technology are fast developing, and the conventional manual suture method for suturing human tissues or organs has been replaced by a method using a medical stapling device and alloy stapling nails. The medical stapling device, which is easy and safe to operate and has minimal trauma, is widely accepted by doctors. Existing stapling nails of medical stapling device is made from titanium alloy. The titanium alloy has high corrosion resistance, and stays in human tissues and organs for a long time, resulting in inflammation and body discomfort. The staple of the stomach tube, the esophagus, or the intestinal canal includes gastrointestinal excision and ends staple so as to rebuild the digestive tract, yet the staple causes wounds of the ends. In addition, the stapling nails used by conventional stapler method for gastrointestinal staple bring negative sequela to human body. Chinese patent CN201320029863.2 disclosed a degradable stapling sleeve made from magnesium alloy for medical use. As shown in FIG. **1**, the degradable stapling sleeve is applicable for the gastrointestinal staple in human body. The stapling sleeve is provided with a plurality of barbs which are opposite arranged. The stapling sleeve is sleeved on ends of two gastrointestinal tubes, and the barbs are configured to hook the tubes, then the sleeve is absorbed by the body as the wound gradually heals up. However, shaping a plurality of barbs on the stapling sleeve having a tube shape is relatively difficult, meanwhile, because the stapling sleeve has only few options of specification and size, usually the stapling sleeve does not completely correspond to the gastrointestinal tubes, thereby limiting the application thereof.

In view of the above-described problems, it is one objective of the invention to provide a degradable surgical staple made from magnesium alloy which is convenient for use and applicable for the stapling of gastrointestinal organs.

The base plate of the staple is provided with a plurality of opposite-distributed and staggered barbs.

When used during surgery, a staple is cut according to the size of the gastrointestinal organs, and optionally, the base plate is rolled inward to form a tube, and the barbs protrude inward from the inner surface of the tube, then the gastrointestinal organ is sheathed in the tube, and hooked by the staggered barbs; or the base plate is rolled outward to form a tube, and the barbs protrude outward from the outer surface of the tube, then the gastrointestinal organ is sleeved on the tube, and hooked by the staggered barbs, thereby facilitating the stapling of gastrointestinal organ.

To achieve the above objective, in accordance with one embodiment of the invention, there is provided a degradable staple made from magnesium alloy for medical use, comprising a base plate made from magnesium alloy. The base plate comprises a surface on which a plurality of opposite-distributed and staggered barbs is disposed.

In a class of this embodiment, the base plate and surfaces of the barbs are coated with a drug layer; the drug layer is absorbable by human body.

In a class of this embodiment, the drug layer is a zinc ion layer. Optionally, the zinc ion layer is replaced by a coating made from biocompatible and corrosion-resistant material, or a coating made from anti-clotting, bactericidal, and bacteriostatic material.

In a class of this embodiment, the base plate having the barbs on the surface operates to roll inward or outward to form a tube for stapling of gastrointestinal organs.

Advantages of the degradable staple according to embodiments of the invention are summarized as follows:

The staple is applicable for the gastrointestinal stapling in human body. In operation, the staple is cut according to the size of the gastrointestinal organs. Optionally, the base plate is rolled inward to form a tube, and the barbs protrude inward from an inner surface of the tube, then two ends of the gastrointestinal organ are sheathed in the tube, slightly pulled, and thus hooked by the staggered barbs; or the base plate is rolled outward to form a tube, and the barbs protrude outward from an outer surface of the tube, then two ends of the gastrointestinal organ are sleeved on the tube, slightly pulled, and thus hooked by the staggered barbs, thereby facilitating the stapling of gastrointestinal organ. The base plate in a tube shape is degraded or absorbed by the human body as the wound gradually heals up.

The base plate and surfaces of the barbs are coated with a drug layer which can be absorbed by the body. The drug layer can be replaced by a coating made from biocompatible and corrosion-resistant material, or a coating made from anti-clotting, bactericidal, and bacteriostatic material. The drug layer and the coating bring no harm to the human body, instead, the drug layer and the coating with strong bactericidal capacity can protect the gastrointestinal organs from infections, thereby shortening the healing time of the wound.

The invention is described hereinbelow with reference to the accompanying drawings, in which:
FIG. **1** is a schematic diagram of a degradable staple made from magnesium alloy for medical use in the prior art;
FIGS. **2A** and **2B** are schematic diagrams of a degradable surgical staple made from magnesium alloy; and
FIGS. **3-5** are diagrams showing stapling of gastrointestinal organs using a degradable surgical staple made from magnesium alloy.

In the drawings, the following reference numbers are used: **1.** Base plate; **2.** Barbs; **3.** Drug layer; **4.** Gastrointestinal organ; **5.** Stapling nails.

For further illustrating the invention, experiments detailing a degradable surgical staple made from magnesium alloy are described below. It should be noted that the following examples are intended to describe and not to limit the invention.

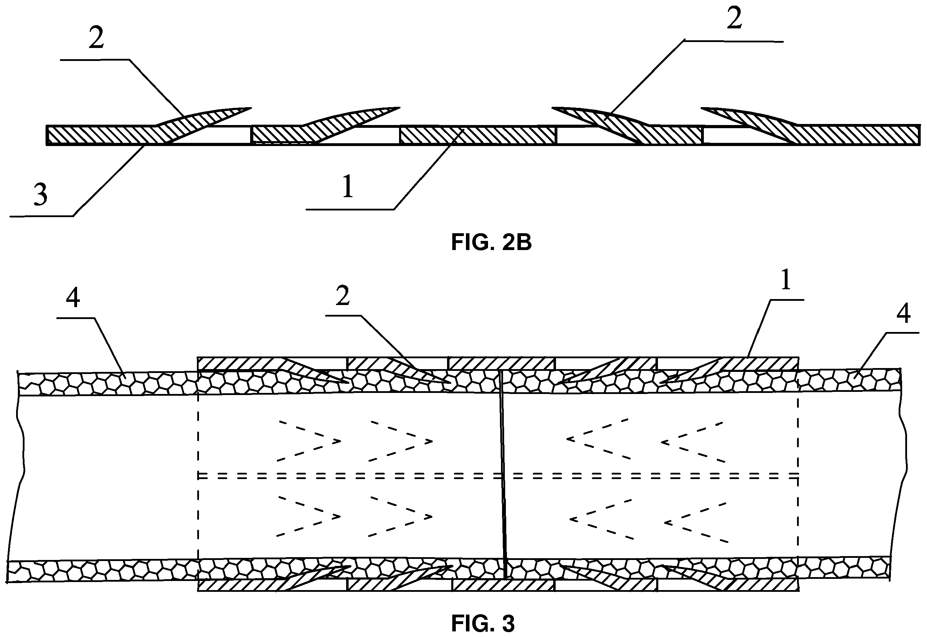

As shown in FIGS. **2A-2B****,** a degradable surgical staple made from magnesium alloy comprises a base plate **1** made from magnesium alloy. The surface of the base plate **1** is provided with a plurality of opposite-distributed and staggered barbs **2.** The base plate **1** and surfaces of the barbs **2** are coated with a drug layer **3.** The drug layer can be absorbed by the body. The base plate **1** having the barbs **2** on the surface operates to roll inward or outward to form a tube for the stapling.

In operation, the base plate **1** of the staple is cut according to the size of the gastrointestinal organs. As shown in FIGS. **3-4****,** the base plate **1** is rolled inward to form a tube, and the barbs **2** protrude inward from an inner surface of the tube, then two ends of the gastrointestinal organ **4** are sheathed in the tube, slightly pulled, and thus hooked by the staggered barbs **2;** or the base plate **1** is rolled outward to form a tube, and the barbs **2** protrude outward from an outer surface of the tube, then two ends of the gastrointestinal organ **4** are sleeved on the tube, slightly pulled, and thus hooked by the staggered barbs **2,** thereby facilitating the stapling of gastrointestinal organ. The base plate in a tube shape is degraded or absorbed by the human body as the wound gradually heals up.

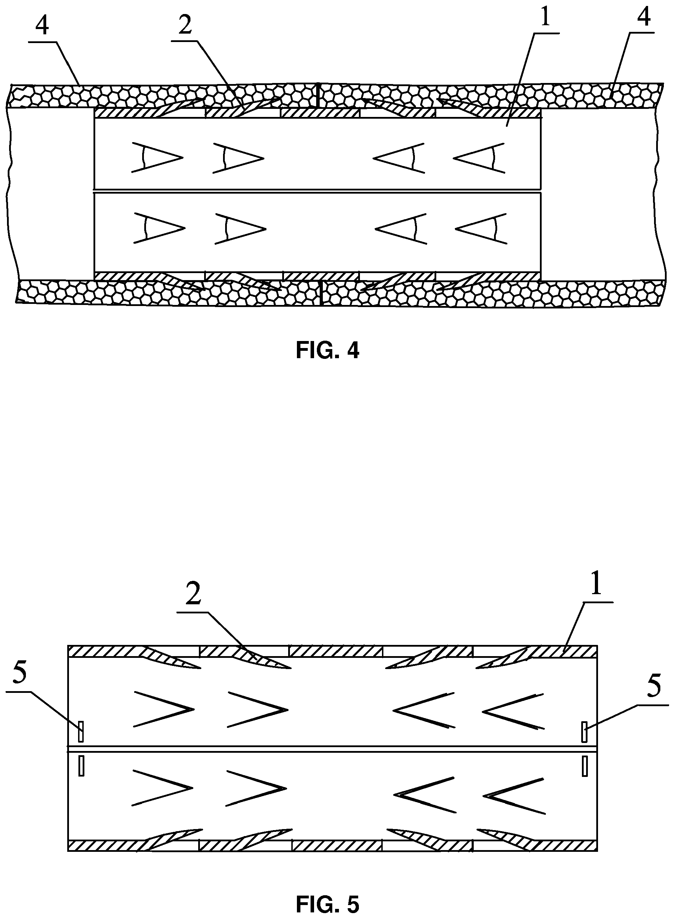

As shown in FIG. **5****,** the stapling of gastrointestinal organ having larger diameters uses the same method, the base plate **1** is also rolled up to form a tube, except that two ends of the tube are fixed by stapling nails **5.**

The base plate **1** and surfaces of the barbs **2** in the invention are coated with the drug layer **3** which can be absorbed by the body. The drug layer can be replaced by the coating made from biocompatible and corrosion-resistant material which facilitates wound healing, or the coating made from anti-clotting, bactericidal, and bacteriostatic material. The drug layer and the coating bring no harm to the human body, instead, the drug layer and the coating with strong anti-clotting property and bactericidal capacity can protect the gastrointestinal organs from infections, thereby shortening the healing time of the wound.

Shaping a plurality of staggered barbs **2** on the base plate **1** is relatively easy. The base plate is rolled up to form a tube so that the staple is applicable for the stapling of gastrointestinal organ, as well as the stapling of esophagus, trachea, artery and vein, achieving a favorable medical stapling as well.

Unless otherwise indicated, the numerical ranges involved in the invention include the end values. While particular embodiments of the invention have been shown and described, it will be obvious to those skilled in the art that changes and modifications may be made without departing from the invention in its broader aspects, and therefore, the aim in the appended claims is to cover all such changes and modifications as fall within the true spirit and scope of the invention.

## Claims

1. A surgical staple, comprising a base plate (1) made from magnesium alloy; **characterized in that** the base plate (1) comprises a surface on which a plurality of opposite-distributed and staggered barbs (2) is disposed.

2. The staple of claim 1, **characterized in that** the base plate (1) and surfaces of the barbs (2) are coated with a drug layer (3); the drug layer is absorbable by human body.

3. The staple of claim 1, **characterized in that** the base plate (1) and surfaces of the barbs (2) are coated with a drug layer (3) which is absorbable by human body, or with a coating made from biocompatible and corrosion-resistant material, or with a coating made from anti-clotting, bactericidal, and bacteriostatic material.

4. The staple of claim 1, **characterized in that** the base plate (1) having the barbs (2) on the surface operates to roll inward to form a tube (4) or outward to form a tube (5) for stapling of gastrointestinal organs.
